## Europäisches Patentamt
## European Patent Office
## Office européen des brevets

(11) Publication number: **0 218 651 B1**

# EUROPEAN PATENT SPECIFICATION

(45) Date of publication of patent specification: **11.08.93**

(51) Int. Cl.5: **C12N 15/16**, C07K 13/00, C12N 15/10, C12N 15/70

(21) Application number: **86902345.7**

(22) Date of filing: **03.04.86**

(86) International application number: **PCT/DK86/00029**

(87) International publication number: **WO 86/05804 (09.10.86 86/22)**

(54) **A DNA SEOUENCE.**

(30) Priority: **03.04.85 DK 1515/85**

(43) Date of publication of application: **22.04.87 Bulletin 87/17**

(45) Publication of the grant of the patent: **11.08.93 Bulletin 93/32**

(84) Designated Contracting States: **AT BE CH DE FR GB IT LI LU NL SE**

(56) References cited:
EP-A-10 009 930
EP-A-10 127 305
EP-A-20 035 384
EP-A-20 115 613

DNA 2 (1983) 265-273

PNAS 80 (1983) 7461-65

(73) Proprietor: **NOVO NORDISK A/S Novo Allé DK-2880 Bagsvaerd(DK)**

(72) Inventor: **ANDERSEN, Henrik, Dalbo /ge Ordrupvej 112 B, 4.tv. DK-2920 Charlottenlund(DK)**
Inventor: **DAHL, Hans, H. M. Birth Defects Research Institute Royal Childrens Hospital Flemington Road Parville, VIC 3052(AU)**

(74) Representative: **von Kreisler, Alek, Dipl.-Chem. et al Patentanwälte, Von Kreisler-Selting-Werner, Postfach 10 22 41, Bahnhofsvorplatz 1 W-5000 Köln 1 (DE)**

**Description**

The present invention concerns a plasmid coding for a mature polypeptide, a process for producing said plasmid, and its use in the production of the desired polypeptide.

The EP Publication 0 001 930 concerns a process for producing a desired polypeptide, e.g. somatostatin, insulin or human growth hormone, comprising the use of a cloning medium expressing a precursor for the said polypeptide, said precursor consisting of the desired polypeptide coupled to another polypeptide so that a selective cleavage site is present between the two polypeptides, enabling enzymatic cleavage so that the desired polypeptide may be isolated. The other polypeptide should preferably have such a size and be so composed as to provide immunogenicity.

The said cloning medium is formed by recombinant techniques, viz. by reading of the heterologous gene coding for the desired polypeptide, copying of the resulting mRNA, isolation of the corresponding cDNA sequence and introduction of it into the cloning medium, e.g. a plasmid.

This method is laborious and also involves the drawback that first a precursor is formed in the expression, having a relatively long pre-sequence to be cleaved to form the mature protein, which may reduce the yield.

An improved method of constructing a replicable cloning medium capable of expressing a desired polypeptide, e.g. human growth hormone, is described in,the DK Patent Application 973/81. This method proceeds from two DNA fragments since e.g. in respect of hGH the DNA sequence of the codons 24-191 is produced by mRNA transcription and the remaining DNA sequence 1-23 by chemical synthesis, and then the two DNA sequences are coupled together and introduced into a plasmid with associated pre-sequence.

Nor is this known process without drawbacks. Thus, the use of the said cloning medium will lead to hGH having the amino acid methionine, which is not present in native hGH, attached to the N-terminal end of the protein. If it is attempted to cleave this methionine group, various mixtures would occur which are difficult to separate by the usual purification methods, and the yield of hGH would be poor.

A solution to the problem of producing hGH in a pure state and a high yield has been proposed in the DK Patent Application 2046/84 disclosing a process which is stated to be able to provide pre-hGH, which may be cleaved to mature hGH, through the use of special plasmids which is used for transformation of the microorganisms E. coli, Pseudomonas aeruginos and Pseudomonas putida. However, practice has shown that the yield of mature hGH is dissatisfactory, and that the correct amino terminal end is not obtained in all cases, so that it has not been possible to isolate native hGH in a pure state by conventional purification methods.

The present invention is based on a heretofore unknown and advantageous plasmid coding for a mature polypeptide having n amino acids coupled with an amino acid extension having m amino acids, **characterized** in that it contains an intermediate comprising a DNA sequence which consists of two coupled DNA fragments, the first of which comprises the coding sequence for the amino acids 1-n of the mature polypeptide, followed by a translation stop signal, preferably a restriction site, and the second DNA fragment, which is coupled to the first DNA fragment at the last base in the first fragment,contains a resistance gene and an origin of replication, said intermediate is ligated to a synthetic linker at codon 1, said linker having an ATG start codon in conjunction with the Cla I restriction site at the 5' end of the + strand, said linker having the sequence

$$\mathrm{CGATG(XX\overset{.}{X})_m}$$
$$\mathrm{TAC(XXX)_m}$$

where X is an arbitrary base, m is 0 or an arbitrary integer, provided that the last base or bases in the linker together with the first base or bases in the intermediate form a restriction site.

Such an intermediate may be introduced into the plasmid in a manner so that the peptide sequence starts with the codon which corresponds to the N-terminal amino acid of the mature protein.

The advantage of this is in particular that there is no need for a unique restriction enzyme cutting site near the N-terminus of the coding sequence for the mature protein.

It has moreover surprisingly been found that it is possible to produce clones by means of the intermediate which code for the desired polypeptide, such as hGH, with pre-sequences which can specifically be cleaved enzymatically in a quantitative yield to provide the pure polypeptide, e.g. hGH, described in the DK Patent Application 556/85 (wo 86/04609).

Thus, according to the invention, it is possible to introduce any desired pre-sequence, and neither the possibility of doing so nor the resulting advantages could be predicted.

As mentioned, the intermediate of the invention is composed of two DNA fragments which may be coupled together through a restriction site. The first of these DNA fragments codes for a desired polypeptide which may in principle be of any type, hGH (with n being 191) or IGF-I (with n being 105) should be emphasized. In addition thereto, other growth hormone types, such as bovine and porcine growth hormone, insulin and somatomedin C, are typical examples of polypeptides which may be expressed by clones which are produced by means of the intermediate.

The coding sequence for the polypeptide in the intermediate of the invention may be produced in several ways, e.g. by reverse transcription of the corresponding mRNA sequence or parts thereof and coupling of these parts. It is also possible to produce the subject DNA sequence by total DNA synthesis. This opens up the possibility of introducing any desired DNA sequence everywhere into the other DNA fragment which is coupled to the coding DNA fragment.

The conversion of the intermediate into a plasmid or another cloning medium and the transformation of a microorganism with the plasmid takes place in a manner known per se.

One of the usual microorganisms, such as E. coli, used for biosynthesis, may serve this purpose.

The invention will be explained and illustrated more fully below with reference to the drawings, in which

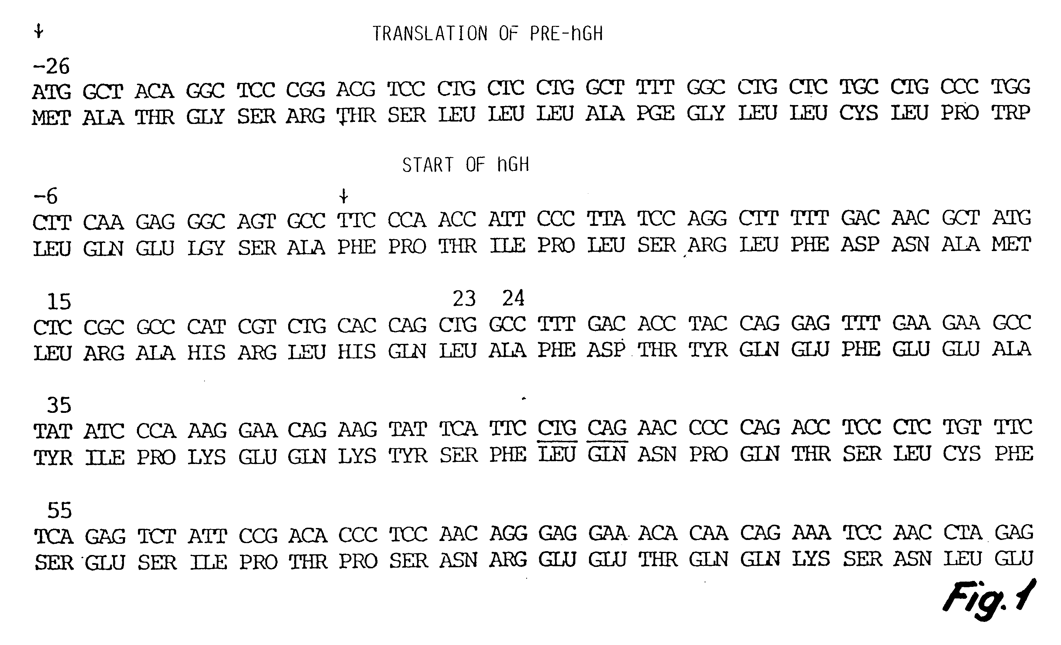

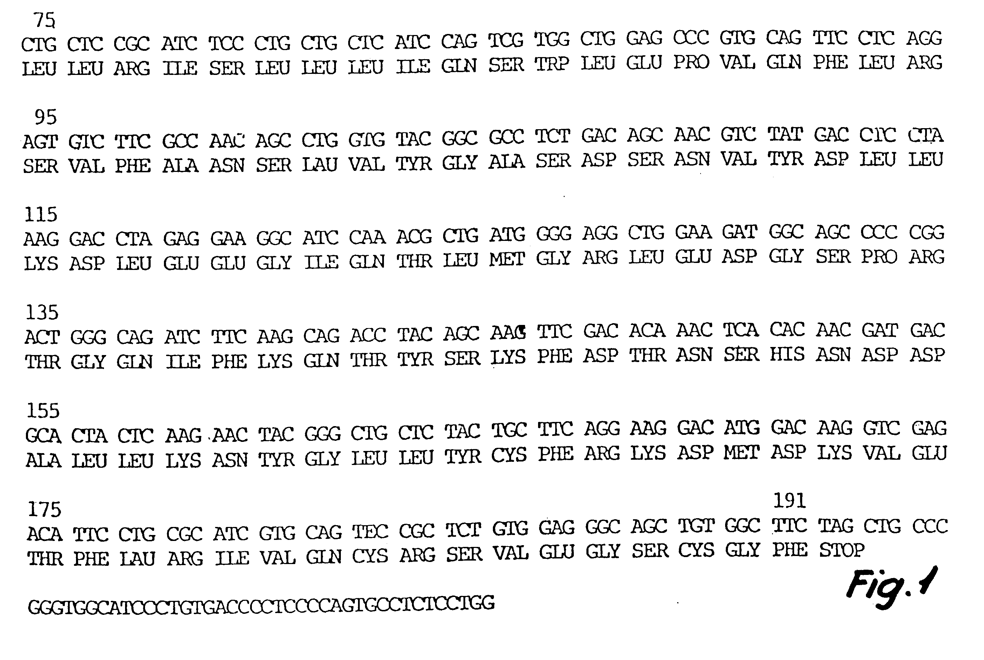

fig. 1 shows the amino acid sequence of pre-hGH together with the associated natural DNA coding sequence;

fig. 2 shows a flow sheet for the construction of a cDNA fragment comprising the coding sequence for the amino aicds 24-191. in hGH and the introduction of it into the plasmid pAT153 to form pH84;

fig. 3 shows a flow sheet for the isolation of a chromosomal 107 bp DNA fragment coding for part of the signal sequence as well as the amino acids 1-23 in hGH;

fig. 4 illustrates the introduction of the 107 bp fragment isolated in accordince with fig. 3 into the plasmid pHB4;

fig. 5 illustrates cutting of pHD80 with Stu 1 and chewing using the Klenow fragment of DNA polymerase I;

fig. 6 shows the formation of the intermediate;

fig. 7 shows the conversion of the intermediate to the clone pHD59-10;

fig. 8 shows an agarose gel with a detailed restriction enzyme analysis of the clones pHD59-7 and pHD59-10;

fig. 9 illustrates a DNA sequence analysis of the clones pHD59-7 and pHD59-10;

fig. 10 shows a flow sheet for cutting of the gene for Met-hGH from pHD59-10, and introduction of it together with the synthetic promotor SP3 and the transcription terminator from the phage fd in the plasmid pAT153 to form pHD67 which is capable of expressing Met-hGH;

figs. 11 and 12 illustrate the construction of an intermediate containing the gene for human IGFI precursor;

fig. 13 shows the use of the intermediate for producing a plasmid coding for Met-IGFI-precursor, and

fig. 14 shows the construction of a plasmid coding for Met-Glu-Ala-Glu-IGFI.

## Construction of an intermeidate containing the gene for hGH

Total RNA was isolated from one or more human hypophyses. The yield was 1-2 mg of RNA/hypophysis. Then 100 $\mu$g of hypophysis RNA were fused with 2 $\mu$g of oligo-(dT), and single stranded cDNA synthesized using reverse transcriptase in a solution containing vanadyl ribonucleoside, ribonuclease inhibitor, 100 mM "Tris" (pH 8.3), 70 mM KCl, 10 mM $MgCl_2$, 10 mM dithiothreitol (DTT), 300 $\mu$M of each dNTP and 100 $\mu$Ci $^{32}$P-dCTP.

The mixture was denatured by heating to 100°C followed by rapid cooling on ice. Then pH was adjusted to 6.9 by addition of Hepes pH 6.9 to 100 mM. Additional 300 $\mu$M of each dNTP and 50 units of DNA polymerase I were added to synthesize the complementary strand. After incubation overnight, the cDNA was fractionated on a "Sephadex® G-50" column, and the first peak was isolated and treated with nuclease SI. Then the cDNA was fractionated on a "Biogel 1.5" column, and the fraction containing cDNA with a length of 400-800 bp was isolated. So-called C tails were applied to this cDNA, which was fused with pBR322 plasmid DNA cut with the restriction enzyme Pst I and then G-tailed. The fused DNA was transformed into Eschericia coli MC1061 in a well-known manner, followed by placing on growth plates containing tetracycline. What corresponds to 10 $\mu$g total RNA gave about 10,000 colonies.

These were hybridized with a probe containing some of the rat growth hormone coding sequence, and 10 positive colonies were selected for further characterization.

DNA restriction enzyme analysis with Hae III showed that one of the clones contained an Hae III fragment of 561 bp, corresponding to the coding sequence for the amino acids (aa) 24-191 in hGH, the translation peak signal and 46 untranslated bases.

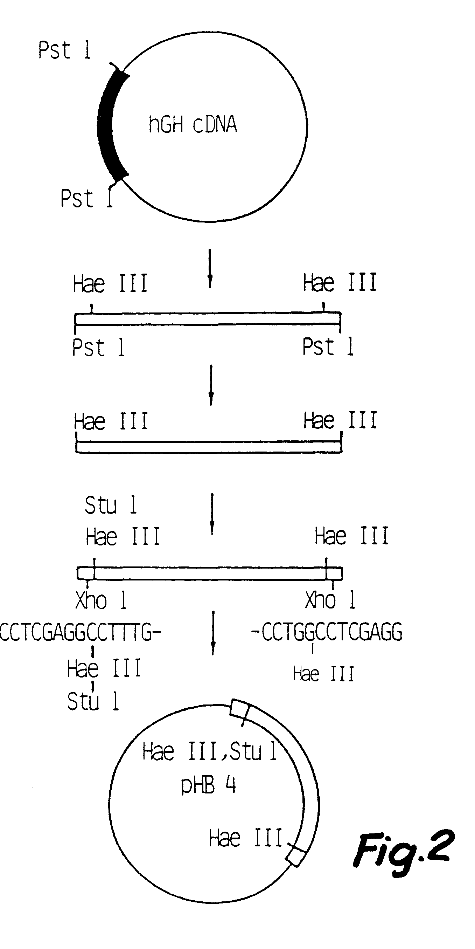

The above-mentioned clone was treated as shown in fig. 2 with a view to construction of full length hGH gene.

First, the cDNA clone is cut with Pst I, and the hGH cDNA fragment is purified on a gel and eluted. The fragment is cut with Hae III, resulting in the above-mentioned fragment which comprises the coding sequence for aa 24-191 in hGH.

To enable later ligation of the missing DNA piece (aa 1-23), it would be expedient if a unique restriction centre is formed at the 5' Hae III centre. This is done by ligating Xho I linkers with the sequence

CCTCGAGG

GGAGCTCC

on the fragment, thereby reforming the Hae III centres (GGCC) at both ends, and at the same time the 5' Hae III centre will likewise be a unique Stu I centre (AGGCCT).

The fragment is then cut with Xho I and may then be ligated into the plasmid pAT153, which has been cut in advance with Sal I since these two centres have the same base overhang:

$$\text{Xho I:} \quad \begin{array}{c} C \mid TCGAG \\ GAGCT \mid C \end{array} \quad \text{Sal I:} \quad \begin{array}{c} G \mid TCGAC \\ CAGCT \mid G \end{array}$$

This results in a plasmid called pH84. It is now possible to open the plasmid with the restriction enzyme Stu I, thereby enabling ligation of the missing DNA piece (aa 1-23).

The ligation mixture was transformed in a known manner into E. coli MC1061, and a plurality of clones were checked for presence of the plasmid pHB4 with the unique Stu I centre. The clone HB4-1, which contained the above-mentioned fragment oriented with the 5' end most adjacent to the unique Eco RI centre of the plasmid, was selected.

## Isolation of a chromosomal DNA fragment coding for the amino acids 13 to 23 in hGH

The phag containing the hGH gene was isolated from a human chromosomal DNA library (again by hybridization with the rat growth hormone DNA), and a 2.6 kb fragment comprising the entire gene was obtained by cutting with Eco RI.

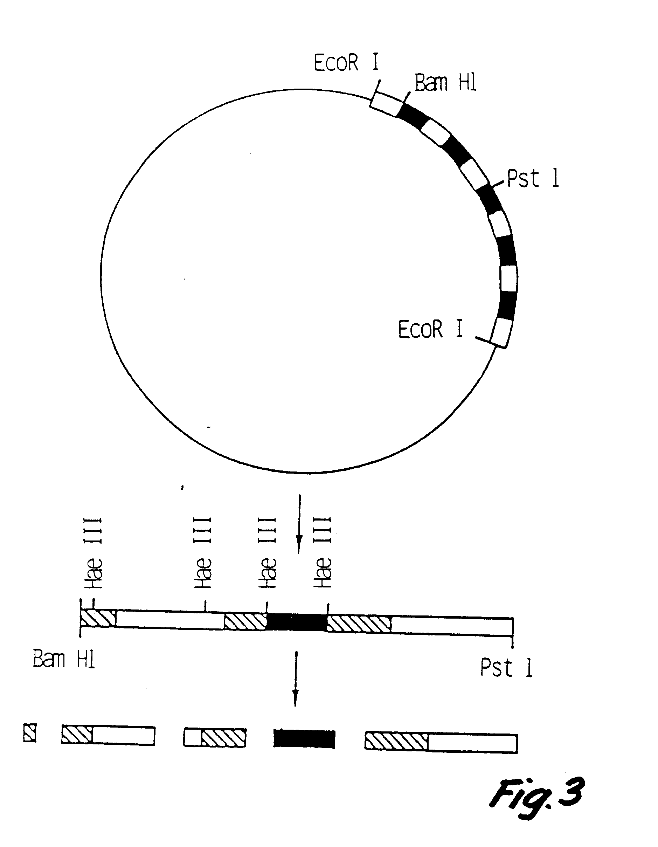

This fragment was treated as shown in fig. 3 with a view to isolating a fragment containing the code for aa-13 to 23 in hGH.

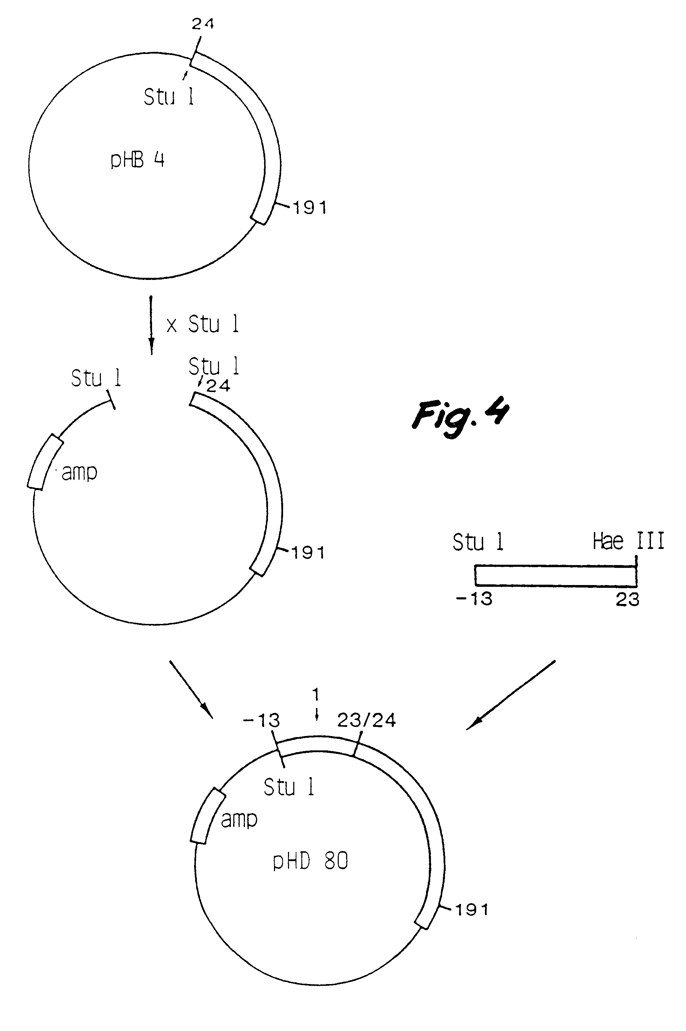

The 2.6 kb fragment is cut with BamHI and Pst I, and a fragment of about 800 bp is isolated on a gel and eluted. The BamHI/Pst I fragment is cut with Hae III, and the fragments are separated according to size on a gel. A 107 bp fragment, which codes for aa 1-23 in hGH as well as 13 amino acids of its natural signal sequence, is isolated and introduced into the plasmid pHB4, cut with Stu I. See fig. 4.

The ligation mixture was transformed in a known manner into E. coli MC1061, and a plurality of clones was checked for presence of the plasmid pHD 80 with the 107 bp fragment positioned in the proper reading frame with respect to the coding sequence for (aa) 24-191.

Thus, in the resulting plasmid, Hae III centres are reformed at both ends, while the 5' Hae III centre likewise constitutes a unique Stu I centre.

It is now possible to open the plasmid with the restriction enzyme Stu I and to treat with nuclease so that cutting will be effected just into the start of the hGH coding sequence.

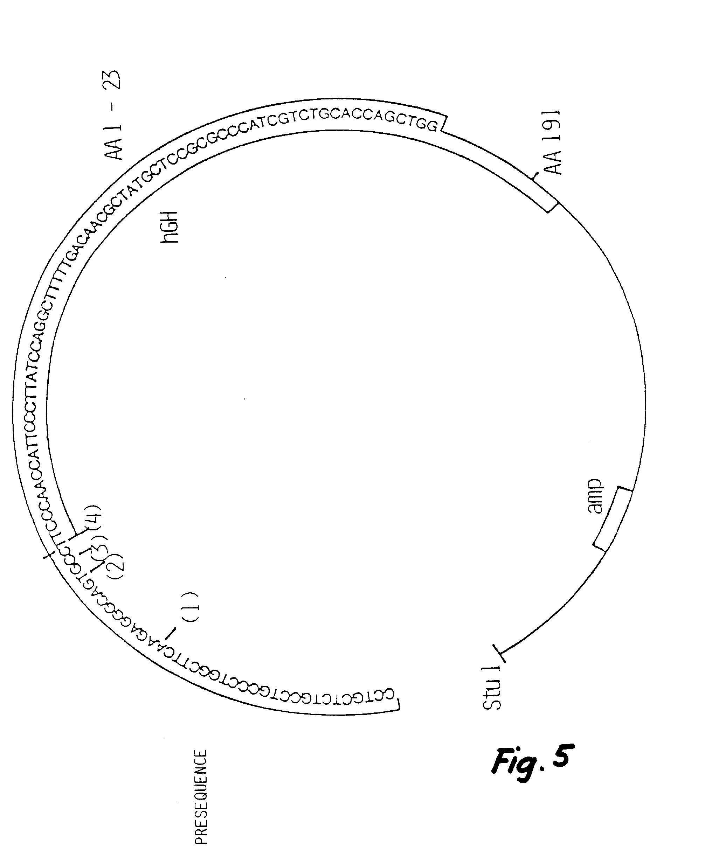

The plasmid pHD80 was cut with Stu I, and chewing was performed as shown in fig. 5 using the Klenow fragment of DNA polymerase I. This enzyme has two activities, a polymerase and an exonuclease activity. Since both activities are dependent upon the presence of nucleotides, it is possible to control the activity of the enzyme by changing the composition of the nucleotides in the reaction mixture.

200 $\mu$g of pHD80 cut with Stu I were used as the starting material. The DNA was treated with Klenow DNA polymerase + dTTP. This causes the DNA molecules to be chewed to the first A in the sequence at point (1). After phenol extraction and ethanol precipitation, the treatment is repeated with Klenow DNA polymerase and dATP, dGTP and $^{32}$P-dATP, respectively, resulting in chewing to (2), (3) and (4), respectively. The plus strand is then removed with SI nuclease.

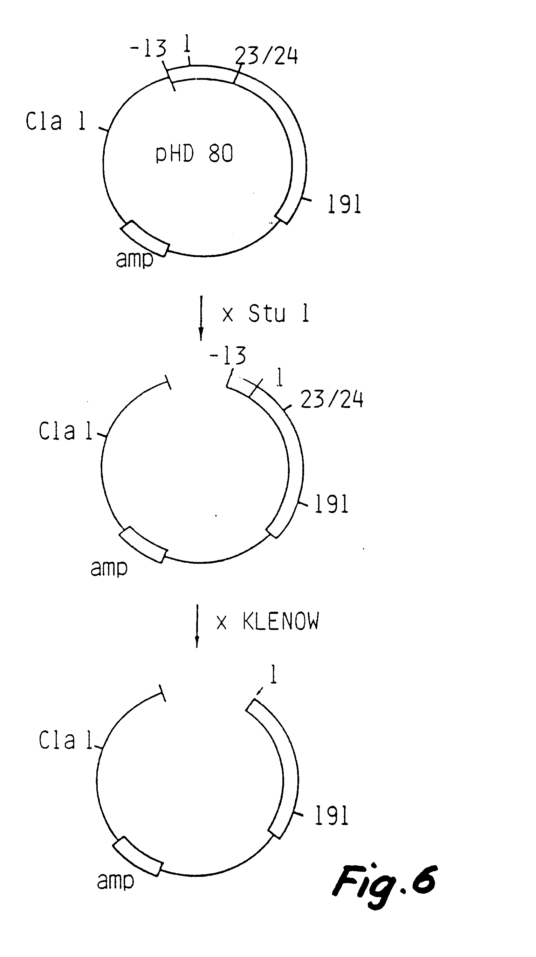

The intermediate (fig. 6) obtained now contains the sequence for hGH and may easily be provided with a synthetic or naturally occurring DNA linker.

When construction any clone produced by ligating the intermediate and a linker having the nucleotide sequence $^{5'}$ GAA$^{3'}$ as the last bases at the 3' end, an Eco RI restriction site

$$5' \quad GAATTC \quad 3'$$
$$GTTAAG$$

will be formed in the transition between the ligated linker and the first codon in the gene for the polypeptide, such as the hGH gen.

This is because the first bases in hGH are

$$5' \quad TTC \quad 3'$$
$$AAG$$

and thus contribute with half of the restriction site. If, on the other hand, the ligated linker contains one of the sequences shown in the following table I, the stated restriction sites will be formed.

Selection of one of the mentioned restriction sites puts a limitation on what the last amino acids coded for by the linker may be. For example, in case of the E. coRI restriction site, coding will take place for xxx Glu-hGH, but this is a desired polypeptide.

Table I

| Sequence at the 3' end of the linker | Sequence at the 5' end of hGH | Formed restriction site |
|---|---|---|
| AAGCT | TTC | Hind III |
| AAGC | TTC | Hind III |
| ACGCG | TTC | MluI |
| AGATC | TTC | BglII - |
| AGGCC | TTC | StuI |
| AGTAC | TTC | ScaI |

The presence of such restriction sites makes it possible to exchange the introduced linker, so that it is not necessary each time to proceed from an intermediate. For example the screening of corrected clones is easier because these may be analysed from the presence of the corrected restriction sites.

Examples of the use of introduced restriction sites between the linker and the hGH gene.

A. Use of the plasmid pHD 86-3 (Met-Ala-Glu-hGH) (example 3) or pDH 86-3SP3 (example 10) as a starting material for construction of clones coding for Met-x-Glu-hGH in which x may be left out or be an arbitrary number of amino acids.

To facilitate exchange of the linker in the plasmid, the Eco RI restriction site which was placed at the 5' end of the promotor was deleted. The deletion was performed by cutting the plasmid partially with Eco RI in the presence of Klenow DNA polymerase and dNTP, causing filling of the restriction site.

Ligation of the DNA and transformation into E. coli MC1061 were followed by selection of a plasmid in which the undesirable Eco RI site was removed.

This plasmid was then cut with Eco RI/Cla I purified on a gel and ligated with the new linkers shown in table II to obtain the stated clones.

Table II

| Clone name | DNA sequence | Amino acid sequence |
|---|---|---|
| pHD 87 | CGATGCTGG<br>TACGACCTTAA | Met-Leu-Glu-hGH |
| pHD 88 | CGATGTTCG<br>TACAAGCTTAA | Met-Phe-Glu-hGH |
| pHD 116 | CGATGGAAGAAG<br>TACCTTCTTCTTAA | Met-Glu-Glu-Glu-hGH |
| pHD 117 | CGATGGAAGCTG<br>TACCTTCGACTTAA | Met-Glu-Ala-Glu-hGH |
| pHD 118 | CGATGACCGAAG<br>TACTGGCTTCTTAA | Met-Thr-Glu-Glu-hGH |
| pHD 119 | CGATGGCTGCTGAAG<br>TACCGACGACTTCTTAA | Met-Ala-Ala-Glu-Glu-hGH |

B. Use of the intermediate to provide a clone coding for Met-Leu-Ala-Val-Ser-hGH

A synthetic linker having the sequence

```
          Met   Leu   Ala   Val   Ser
    CG    ATG   CTG   GCT   GTA   AGC
          TAC   GAC   CGA   CAT   TCG
```

was ligated to the intermediate, which was cut with Cla I, purified on a gel ligated and transformed into E. coli MC1061.

It will now be possible to cut this clone with Cla I/Hind III since a Hind III restriction site has been formed between the linker and the hGH gene.

Any linker having the sequence

```
    CG    ATG  (xxx)n  xyA
          TAC  (xxx)n  xzTTCGA
```

may now be introduced.

X is an arbitrary base and n is 0 or arbitrary and Y and Z are ≠ A and T, respectively.

Construction of an intermediate coding for human IGFI precursor

Clones containing IGF I-cDNA are isolated from a human liver cDNA library constructed in λGT 11, by hybridization with an IGF-I specific synthetic DNA fragment.

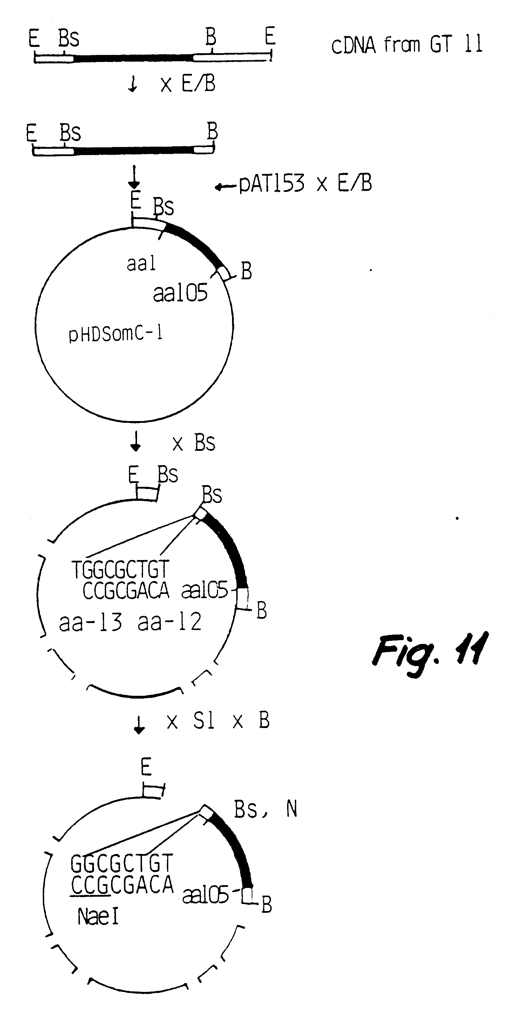

DNA is isolated from the clones and is cut with the restriction enzymes ECoRI/BamHI. The DNA is fractionated on a gel, and a clone containing a fragment greater than 400 bp is selected. DNA from this clone is cut with Eco RI/BamHI. A fragment of about 400 bp is isolated and introduced into the plasmid pAT153 cut with EcoRI/BamHI (fig. 11A).

The ligation mixture is transformed in a known manner into an E. coli MC1061, and a plurality of clones is checked for presence of the fragment.

The formed plasmid (pHD Som C-1) is now cut with the restriction enzyme BstNI and treated with Sl nuclease so that the base overhang is cut away. This causes formation of an NaeI restriction site at one end of the IGF I fragment (see fig. 11B). The DNA is phenolated, precipitated and cut with the restriction enzyme BamHI (fig. 11C).

A fragment of about 400 base pairs is isolated and introduced into the plasmid pBR 322 cut with NaeI/BamHI.

The ligation mixture is transformed in a known manner into E. coli MC1061 and a plurality of clones is checked for presence of the 400 bp NaeI/BamHI fragment.

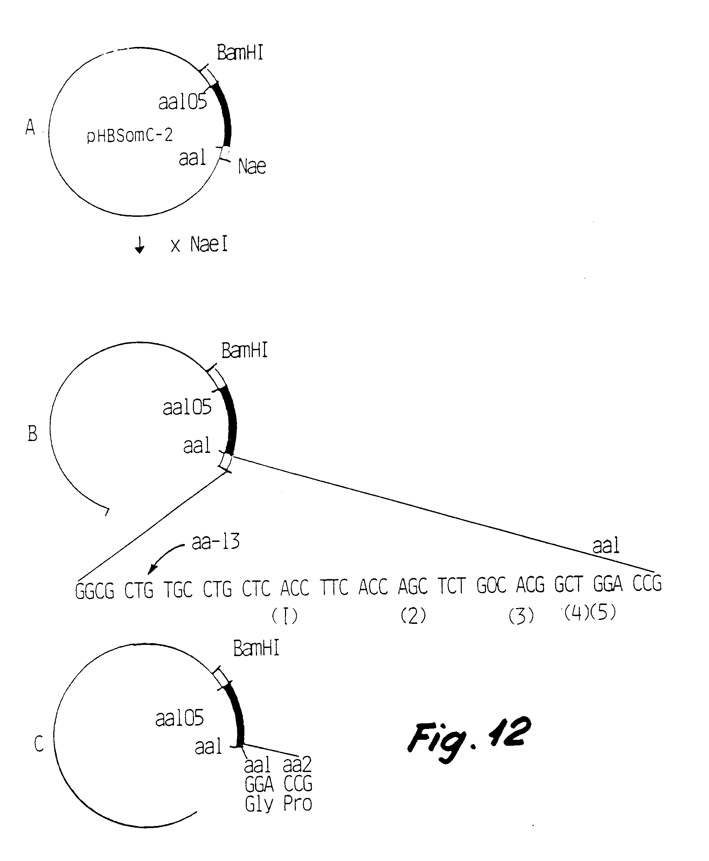

The formed plasmid pHD Som C-2 (fig. 12A) is now cut with the restriction enzyme NaeI and treated with nuclease so that cutting takes place just down to the start of the IGF I coding sequence.

pHD Som C-2 was cut with NaeI, and chewing was performed as shown in fig. 12B using the Klenow fragment from DNA polymerase I.

200 $\mu$g of pHD Som C-2 were treated with Klenow DNA polymerase + dTTP. This causes the DNA, molecule to be chewed down to the first A in the sequence at point (1). After phenol extraction and ethanol precipitation the treatment with Klenow DNA polymerase and dCTP, dTTP, dATP and dCTP, respectively, is repeated, causing chewing to (2), (3), (4) and (5), respectively. The plus strand is then removed with Sl nuclease. The resulting intermediate (fig. 12C) may now easily be ligated with a synthetic or naturally occurring DNA linker.

## EXAMPLE 1

Use of an intermediate for the construction of a plasmid coding for met-hGH

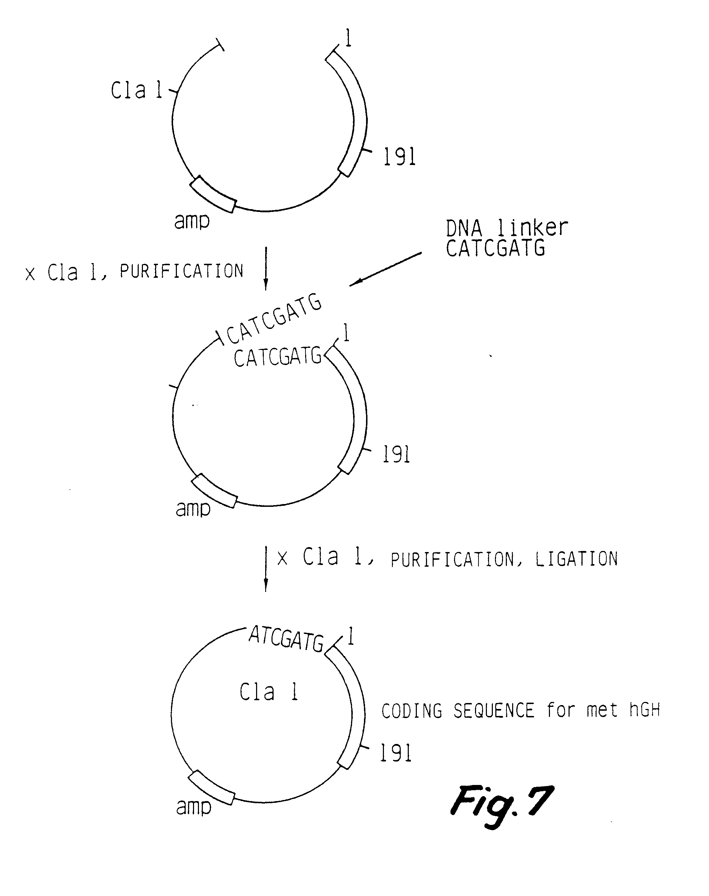

The translation start signal is ATG which codes for the amino acid methionine. Since ClaI linkers contain precisely this sequence together with a ClaI centre, ClaI linker was ligated on the intermediate as shown in fig. 7. The DNA was then cut with ClaI, purified on a gel and ligated.

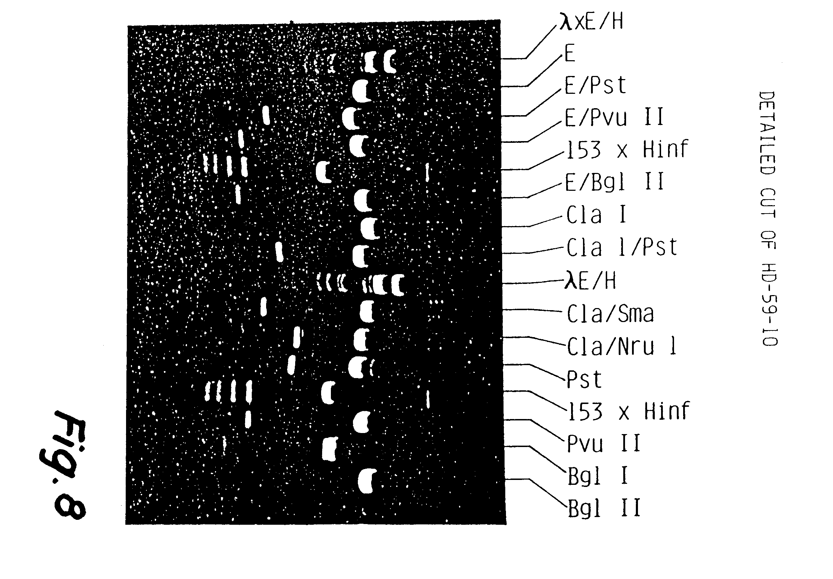

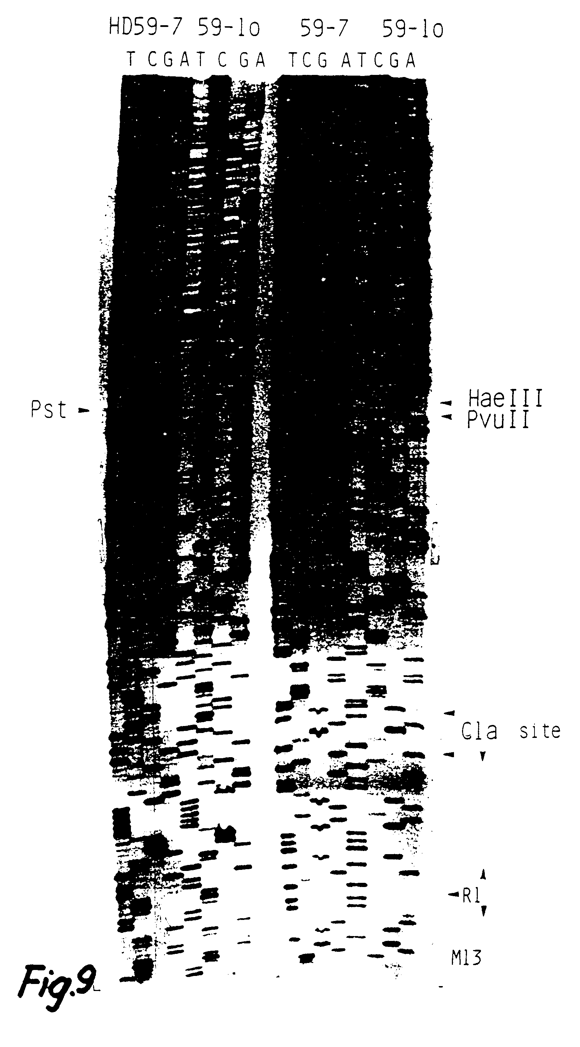

The ligation mixture was transformed in a known manner into E. coli MC1061, and 24 clones were isolated, and DNA from each of these was analysed by means of restriction enzyme cuts. 6 of the clones showed the expected restriction pattern when cut with the enzymes ClaI/PvuII. 2 clones, DH59-7 and HD59-10, were selected for further analysis by means of DNA sequence determination. The sequence analysis showed that they had ClaI linker introduced at the correct position and thus an ATG start codon in the same reading frame as in hGH and contained the coding sequence for Met-hGH (fig. 9). HD59-10 was additionally characterized by detailed restriction enzyme analysis (fig. 8).

Thus, a DNA sequence coding for Met-hGH has been constructed.

## EXAMPLE 2

Use of an intermediate for construction of a plasmid coding for met-phe-glu-glu-hGH

A synthetic linker having the sequence

CGATGTTCGAAGAA

TACAAGCTTCTT

was ligated on the intermediate. The resulting DNA fragment was then cut with ClaI , purified on a gel and ligated.

The ligation mixture was transformed in a known manner into E. coli MC1061, and 6 clones were isolated. DNA from each of these was isolated and analysed by restriction enzyme cuts. 4 showed the expected pattern. 2 clones, pHD65-4 and pHD65-5, were selected for further analysis by means of DNA sequence determination.

The sequence analysis showed that they had the linker introduced in the same reading frame as in hGH and contained the coding sequence for met-phe-glu-glu-hGH.

EXAMPLE 3

Use of the intermediate for construction of a plasmid coding for met-ala-glu-hGH

A synthetic linker having the sequence

$$CGATGGCTGAA$$
$$TACCGACTT$$

was ligated on the intermediate, which was cut with ClaI, purified, ligated and transformed into E. coli MC1061, as stated in example 2.

The clones pHD86-2 and pHD86-3 were selected, which were shown by sequence analysis to contain the coding sequence for met-ala-glu-hGH.

EXAMPLE 4

Use of an intermediate for construction of a plasmid coding for met-ala-glu-ala-glu-hGH

A synthetic linker having the sequence

$$CGATGGCTGAAGCTGAA$$
$$TACCGACTTCGACTT$$

was ligated on the intermediate, which was cut with ClaI, purified, ligated and transformed into E. coli MC1061, as stated in example 2.

The clones pHD107-1 and pHD107-4 were selected, which were shown by sequence analysis to contain the coding sequence for met-ala-glu-ala-glu-hGH.

EXAMPLE 5

Use of an intermediate for construction of a plasmid coding for met-ala-glu-glu-hGH

A synthetic linker having the sequence

$$CGATGGCTGAAGAA$$
$$TACCGACTTCTT$$

was ligated on the intermediate, which was cut with ClaI, purified, ligated and transformed into E. coli MC1061, as stated in example 2.

The clones pHD108-1 and pHD108-2 were selected, which were shown by sequence analysis to contain the coding sequence for met-ala-glu-glu-hGH.

EXAMPLE 6

Use of an intermediate for construction of a plasmid coding for Met-IGF-I-Precursor

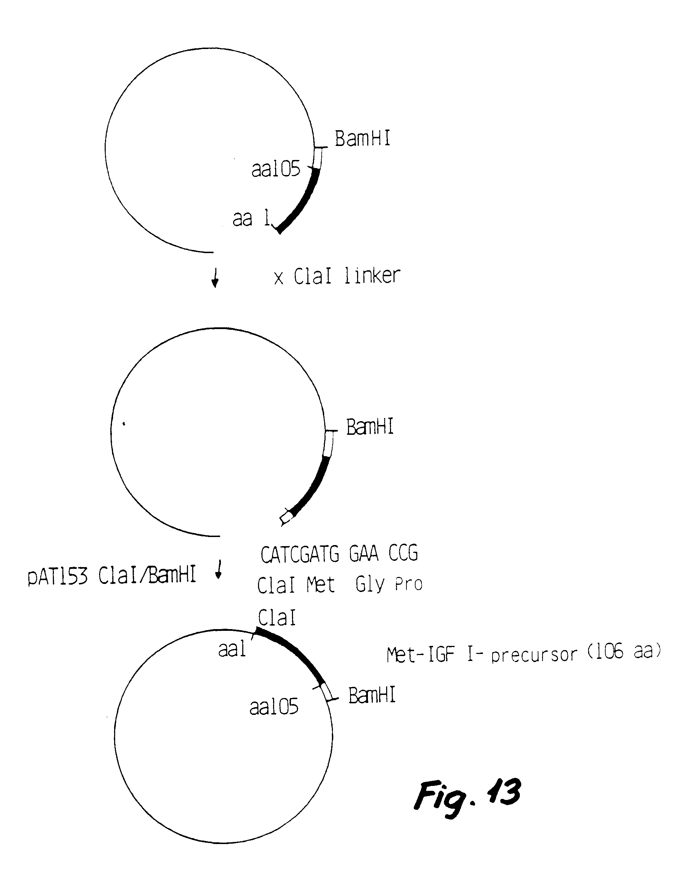

The translation start signal is ATG which codes for the amino acid methionine. Since ClaI linkers contain precisely this sequence together with a ClaI restriction centre, ClaI linkers are ligated on the intermediate, as shown in fig. 13.

8

The DNA was then cut with ClaI/BamHI. A DNA fragment of about 355 bp was purified on a gel and introduced into the plasmid pAT153 cut with ClaI/BamHI.

Thus, a DNA sequence coding for Met-IGF-I-Precursor has been constructed.

EXAMPLE 7

Use of an intermediate for construction of a plasmid coding for Met-Glu-Ala-Glu-IGF-I-Precursor

A synthetic linker having the sequence

```
CG   ATG   GAA   GCT   GAA
     TAC   CTT   CGA   GTT
```

was ligated on the intermediate. The obtained DNA was cut with ClaI/BamHI. A DNA fragment of about 360 bp was purified on a gel and introduced into the plasmid pAT153 cut with ClaI/BamHI.

Thus, a DNA sequence coding for Met-Glu-Ala-Glu-IGF-I-Precursor has been constructed.

EXAMPLE 8

May be performed along the above-mentioned pattern with the synthetic sequences used in the patent for hGH.

Expression of biosynthetic hGH

All the foregoing plasmids have been constructed with a view to cloning the introduced gene fragments by replication of the plasmid in a microorganism transformed with it and expression of the desired protein. However, expression of a eukaryotic gene in microorganisms requires a replicable expression medium containing a promotor.

The purpose of the promotor is to bind RNA polymerase and thus start the transcription of the gene or genes which are subordinated to the promotor in question. However, in addition to the promotor, also a so-called Shine Dalgarno sequence is necessary, which is a region on the mRNA which is responsible for its binding to the ribosomes and thus its translation to protein.

EXAMPLE 9

Expression of Met-hGH

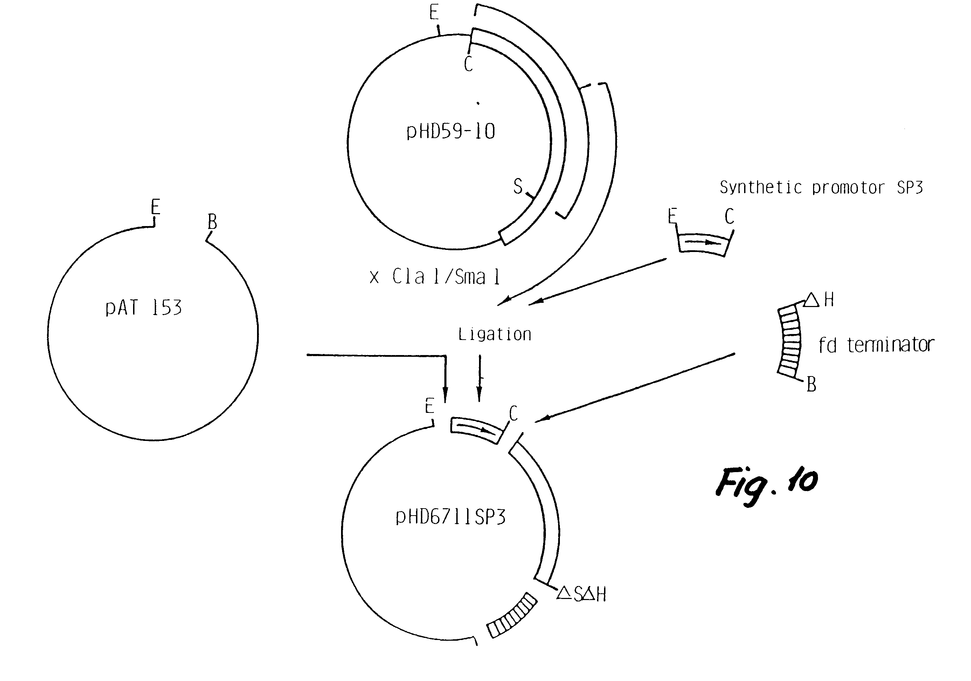

A plasmid expressing Met-hGH under control of a synthetic promotor was constructed using the strategy shown in fig. 10;

A fragment containing the Met-hGH coding sequence and the translation stop signal as well as 2 bases 5' pre- and 6 untranslated bases 3' for the hGH sequence was cut out of the plasmid pHD59-10 with the restriction enzymes Cla I/Sma I and isolated by gel electrophoresis.

A synthetic DNA fragment was constructed, containing EcoRI centre, RNA polymerase binding centre, ribosome binding centre and Cla I centre. This promotor SP3 has the nucleotide sequence stated by the + strand:

```
GAATTCGATCCTGTTGACAATTAATCATAGAGCTCGTA
  TAATGTGGAATTGTGCAGATCTAACAATTAAGCTT
    AGGATCTAGAAATCGAT
```

It is known from the literature that a transcription terminator, introduced after translation stop in the gene to be expressed, is an advantage since the terminator when stopping the transcription prevents reading of and thus interference with other genes, regulation areas and the start area for DNA replication. Such a terminator may e.g. be the terminator from the phage fd.

An about 375 bp Hind III fragment containing the fd terminator was isolated and made blunt ended by means of Klenow polymerase + dNTP. The fragment was then cut with the restriction enzyme BamHI, and an about 365 bp fragment with a blunt 5' end and a BamHI overhang at the 3' end was isolated.

The above-mentioned three isolated fragments were then ligated into the plasmid pAT153, which was cut with EcoRI/BamHI. Thus, the hGH production was subordinated to the synthetic promotor. The plasmid thus constructed was called pHD67.

The ligation mixture was transformed in a known manner into E. coli MC1061. The clone HD6711SP3 was selected and cultivated in 10 ml of LB medium admixted with ampicillin. After 8 hours' growth, the cells were harvested, lysed by ultrasound treatment and analysed for content of hGH immuno-reacting peptides by means of ELISA and RIA.

A comparison of the RIA and ELISA results between hypophyseal hGH, Nanormon ® and Met-hGH produced by cultivation of HD6711SP3 showed that they thinned in parallel. Cell lysate from HD6711SP3 was run directly on HPLC as pure cell lysate as well as cell lysate mixed with Nanormon ®. A peak characteristic of hGH occurred in both cases. The first 15 amino acids were determined by automatic Edman degradation after purification of the product (P. Edman and C. Begg, Eur. J. Biochem. 1 (1967), page 80-91) and found to be identical with the amino acid sequence for Met-hGH.

The biological activity of the produced bacterial Met-hGH was examined by a tibia test and found to correspond to the activity of Nanormon ®.

EXAMPLE 10

Expression of met-ala-glu, met-phe-glu-glu, met-ala-glu-ala-glu and met-ala-glu-glu

The method is performed in the same manner as described for Met-hGH in example 9.

EXAMPLE 11

Expression of Met-IGF-I-Precursor

A plasmid expressing Met-IGF-I-Precursor under control of a synthetic promotor was constructed.

The fragment containing the Met-IGF-I-Precursor gene was isolated by cutting with ClaI/BamHI. The fragment was introduced into a plasmid with a synthetic promotor and ribosome binding site and transcription terminator in principle as described in example 9.

EXAMPLE 12

Expression of Met-Glu-Ala-Glu-IGF-I-Precursor

The method is performed as described in claim 11. May be repeated with the other amino terminal extended IGF-I-Precursors.

EXAMPLE 13

Use of the Met-Glu-Ala-Glu-IGF-I precursor plasmid for construction of a plasmid coding for Met-Glu-Ala-Glu-IGF I

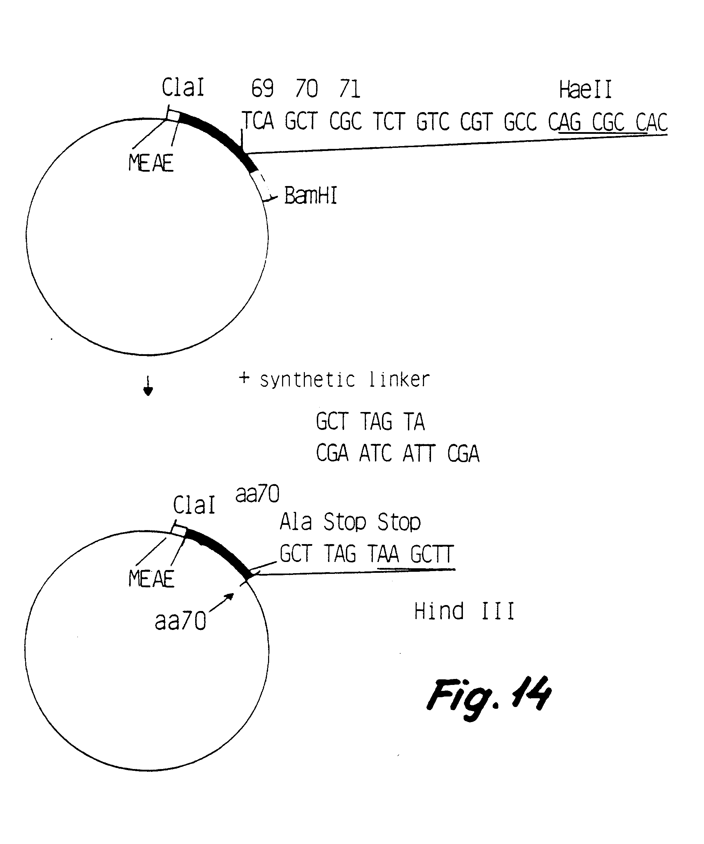

The plasmid containing the coding sequence for the Met-Glu-Ala-Glu-IGF-I precursor was cut with the restriction enzyme Hae II fig. 14 and treated with Klenow DNA polymerase to remove the coding sequence for the 3' precursor fragment (amino acid (aa) 71-105).

200 $\mu$g of DNA were treated with Klenow DNA polymerase + dTTP. This caused chewing to point (1) in fig. 14. After phenolation and ethanol precipitation, the Klenow DNA polymerase and dATP treatment was repeated, causing chewing to point (2).

The minus strand is then removed with SI nuclease. The resulting product is ligated with a synthetic DNA linker having the sequence

GCTTAGTA

CGAATCATTCGA

Then cutting with the restriction enzyme ClaI is performed. A fragment of about 210 bp is purified on a gel and is introduced into the plasmid pAT153, cut with ClaI/Hind III. Thus, a plasmid containing the coding sequence for Met-Glu-Ala-Glu-IGF I (70aa) has been constructed.

EXAMPLE 14

Expression of Met-Glu-Ala-Glu-IGF I

A plasmid expressing Met-Glu-Ala-Glu-IGF I under control of a synthetic promotor was constructed by introducing the fragment having the coding sequence for Met-Glu-Ala-Glu-IGF I into an expression plasmid containing a promotor and synthetic ribosome binding site as well as optionally transcription terminator in principle as described in example 11.

The plasmid was introduced into E. coli MC1061 in a known manner.

The clone pHD Som C-56SP13 was selected and cultivated in 10 ml of LB medium admixed with ampicillin.

After 8 hours' growth, the cells were harvested and lysed by ultrasound treatment and analysed for content of IGF I immunoreacting peptides by means of ELISA.

**Claims**
**Claims for the following Contracting States : DE, NL, GB, CH, LI, FR, LU, SE, BE, IT**

1.  A plasmid coding for a mature polypeptide having n amino acids coupled with an amino acid extension having m amino acids, **characterized** in that it contains an intermediate comprising a DNA sequence which consists of two coupled DNA fragments, the first of which comprises the coding sequence for the amino acids 1-n of the mature polypeptide, followed by a translation stop signal, preferably a restriction site, and the second DNA fragment, which is coupled to the first DNA fragment at the last base in the first fragment, contains a resistance gene and an origin of replication, said intermediate is ligated to a synthetic linker at codon 1, said linker having an ATG start codon in conjunction with the Cla I restriction site at the 5' end of the + strand, said linker having the sequence

$$CGATG(XXX)_m$$
$$TAC(XXX)_m$$

where X is an arbitrary base, m is 0 or an arbitrary integer, provided that the last base or bases in the linker together with the first base or bases in the intermediate form a restriction site.

2.  A plasmid according to claim 1, **characterized** in that the first DNA fragment of the intermediate has the coding properties characteristic of hGH, with n being 191.

3.  A plasmid according to claim 1, **characterized** in that the first DNA fragment of the intermediate has the coding properties characteristic of the precursor of IGF-I with n being 105.

4.  A process for producing a plasmid coding for a mature polypeptide having n amino acids coupled with an amino acid extension having m amino acids, **characterized** in that an intermediate of the composition defined in claim 1 is ligated at codon 1 to a synthetic linker having an ATG start codon in conjunction with a Cla I restriction site at the 5' end of the + strand, said synthetic linker having the sequence

$$CGATG(XXX)_m$$
$$TAC(XXX)_m$$

where X is an arbitrary base, m is 0 or an integer, and the bases are preferably selected in such a way that the first base or bases in the linker together with the first base or bases in the intermediate form a restriction site.

5. A process according to claim 4, **characterized** in that also a suitable promotor and ribosome binding site are ligated into said plasmid.

6. Use of the plasmid produced according to claim 4 or 5 for introduction into a microorganism, such as E. coli, and fermentation on a substrate in order to produce the desired polypeptide.

**Claims for the following Contracting State : AT**

1. A process for producing a plasmid coding for a mature polypeptide having n amino acids coupled with an amino acid extension having m amino acids, wherein said plasmid contains an intermediate comprising a DNA sequence which consists of two coupled DNA fragments, the first of which comprises the coding sequence for the amino acids 1-n of the mature polypeptide, followed by a translation stop signal, preferably a restriction site, and the second DNA fragment, which is coupled to the first DNA fragment at the last base in the first fragment, contains a resistance gene and an origin of replication characterized in that said intermediate is ligated to a synthetic linker at codon 1, said linker having an ATG start codon in conjunction with the Cla I restriction site at the 5' end of the + strand, said linker having the sequence

$$CGATG(XXX)_m$$
$$TAC(XXX)_m$$

where X is an arbitrary base, m is 0 or an arbitrary integer, provided that the last base or bases in the linker together with the first base or bases in the intermediate form a restriction site.

2. A process according to claim 1, **characterized** in that the first DNA fragment of the intermediate has the coding properties characteristic of hGH, with n being 191.

3. A process according to claim 1, **characterized** in that the first DNA fragment of the intermediate has the coding properties characteristic of the precursor of IGF-I with n being 105.

4. A process according to any of the claims 1-3 **characterized** in that also a suitable promotor and ribosome binding site are ligated into said plasmid.

5. Use of the plasmid produced according to any one of claims 1-4 for introduction into a microorganism, such as E. coli, and fermentation on a substrate in order to produce the desired polypeptide.

**Patentansprüche**

**Patentansprüche für folgende Verstragsstaaten : DE, NL, GB, CH, LI, FR, LU, SE, BE, IT**

1. Plasmid, welches für ein reifes Polypeptid mit n Aminosäuren, verknüpft mit einer Aminosäurenverlängerung mit m Aminosäuren, codiert, dadurch gekennzeichnet, daß es ein Intermediat enthält, das eine DNA-Sequenz umfaßt, welche aus zwei verknüpften DNA-Fragmenten besteht, von denen das erste die Codiersequenz für die Aminosäuren 1-n des reifen Polypeptids, gefolgt von einem Translations-Stopsignal, vorzugsweise eine Restriktionsstelle, umfaßt, und das zweite DNA-Fragment, welches mit dem ersten DNA-Fragment an der letzten Base in dem ersten Fragment verknüpft ist, ein Resistenzgen und einen Replikationsursprung besitzt, das Intermediat mit einem synthetischen Linker am Codon 1 ligasiert ist, der Linker ein ATG-Startcodon in Konjugation mit der Cla I Restriktionsstelle an dem 5' Ende des +Stranges aufweist, der Linker die Sequenz

$$CGATG(XXX)_m$$
$$TAC(XXX)_m$$

aufweist, worin X eine beliebige Base ist, m 0 oder eine beliebige ganze Zahl ist, vorausgesetzt, daß die letzte Base oder Basen in dem Linker zusammen mit der ersten Base oder Basen in dem Intermediat eine Restriktionsstelle bilden.

2. Plasmid gemäß Anspruch 1, dadurch gekennzeichnet, daß das erste DNA-Fragment des Intermediates die für hGH, mit n = 191, charakteristischen Codiereigenschaften aufweist.

3. Plasmid gemäß Anspruch 1, dadurch gekennzeichnet, daß das erste DNA-Fragment des Intermediates die für den Vorläufer von IGF-I, mit n = 105, charakteristischen Codiereigenschaften aufweist.

4. Verfahren zum Herstellen eines Plasmids, welches für ein reifes Polypeptid mit n Aminosäuren, verknüpft mit einer Aminosäureverlängerung mit m Aminosäuren, codiert, dadurch gekennzeichnet, daß ein Intermediat der in Anspruch 1 definierten Zusammensetzung am Codon 1 mit einem synthetischen Linker ligasiert wird, der ein ATG-Startcodon in Konjugation mit einer Cla I Restriktionsstelle an dem 5' Ende des +Stranges aufweist, der genannte synthetische Linker die Sequenz

$$CGATG(XXX)_m$$
$$TAC(XXX)_m$$

aufweist, worin X eine beliebige Base, m 0 oder eine ganze Zahl ist und die Basen vorzugsweise dergestalt ausgewählt sind, daß die erste Base oder Basen in dem Linker zusammen mit der ersten Base oder Basen in dem Intermediat eine Restriktionsstelle bilden.

5. Verfahren gemäß Anspruch 4, dadurch gekennzeichnet, daß ebenfalls ein geeigneter Promotor und eine Ribosomen-Bindungsstelle in das Plasmid ligasiert sind.

6. Verwendung des gemäß Anspruch 4 oder 5 hergestellten Plasmids zur Einführung in einen Mikroorganismus, wie E. coli, und Fermentation auf einem Substrat, um das erwünschte Polypeptid herzustellen.

**Patentansprüche für folgenden Vertragsstaat : AT**

1. Verfahren zum Herstellen eines Plasmids, welches für ein reifes Polypeptid mit n Aminosäuren, verknüpft mit einer Aminosäurenverlängerung mit m Aminosäuren, codiert, wobei das genannte Plasmid ein Intermediat enthält, welches eine DNA-Sequenz umfaßt, die aus zwei verknüpften DNA-Fragmenten besteht, von denen das erste die die Codiersequenz für die Aminosäuren 1-n des reifen Polypeptids, gefolgt von einem Translations-Stopsignal, vorzugsweise eine Restriktionsstelle, umfaßt und das zweite DNA-Fragment, welches mit dem ersten DNA-Fragment an der letzten Base des ersten Fragmentes verknüpft ist, ein Resistenzgen und einen Replikationsursprung enthält, dadurch gekennzeichnet, daß das Intermediat mit einem synthetischen Linker am Codon 1 ligasiert ist, der Linker ein ATG-Startcodon in Konjugation mit der Cla I Restriktionsstelle an dem 5' Ende des +Stranges aufweist, der genannte Linker die Sequenz

$$CGATG(XXX)_m$$
$$TAC(XXX)_m$$

aufweist, worin X eine beliebige Base ist, m 0 oder eine beliebige Zahl ist, vorausgesetzt, daß die letzte Base oder Basen in dem Linker zusammen mit der ersten Base oder Basen in dem Intermediat eine Restriktionsstelle bilden.

2.  Verfahren gemäß Anspruch 1, dadurch gekennzeichnet, daß das erste DNA-Fragment des Intermediates die für hGH, mit n = 191, charakteristischen Codiereigenschaften aufweist.

3.  Verfahren gemäß Anspruch 1, dadurch gekennzeichnet, daß das erste DNA-Fragment des Intermediates die für den Vorläufer von IGF-I, mit n = 105, charakteristischen Codiereigenschaften aufweist.

4.  Verfahren gemäß irgendeinem der Ansprüche 1-3, dadurdch gekennzeichnet, daß ebenfalls ein geeigneter Promotor und eine Ribosomen-Bindungsstelle in das Plasmid ligasiert sind.

5.  Verwendung des gemäß irgendeinem der Ansprüche 1-4 hergestellten Plasmids für die Einfügung in einen Mikroorganismus, wie E. coli, und Fermentation auf einem Substrat, um das erwünschte Polypeptid herzustellen.

**Revendications**
**Revendications pour les Etats contractants suivants : DE, NL, GB, CH, LI, FR, LU, SE, BE, IT**

1.  Plasmide codant pour un polypeptide mature possédant n amino-acides couplés à une séquence d'allongement d'amino-acides possédant m amino-acides, caractérisé en ce qu'il contient un intermédiaire comprenant une séquence d'ADN qui consiste en deux fragments d'ADN couplés, dont le premier comprend la séquence codant pour les amino-acides 1 à n du polypeptide mature, suivie par un signal de terminaison de traduction, de préférence un site de restriction, et dont le second fragment d'ADN, qui est couplé au premier fragment d'ADN au niveau de la dernière base dans le premier fragment, contient un gène de résistance et une origine de réplication, ledit intermédiaire étant réuni par ligation à un segment synthétique de liaison au niveau du codon 1, ledit segment de liaison possédant un codon d'initiation ATG conjointement avec le site de restriction Cla I à l'extrémité 5' du brin positif, ledit segment de liaison ayant la séquence

$$CGATG(XXX)_m$$
$$TAC(XXX)_m$$

dans laquelle X représente une base arbitraire, m est égal à 0 ou à un nombre entier arbitraire, sous réserve que la ou les dernières bases dans le segment de liaison conjointement avec la ou les premières bases dans l'intermédiaire forment un site de restriction.

2.  Plasmide suivant la revendication 1, caractérisé en ce que le premier fragment d'ADN de l'intermédiaire possède les propriétés de codage caractéristiques de la hGH, n étant égal à 191.

3.  Plasmide suivant la revendication 1, caractérisé en ce que le premier fragment d'ADN de l'intermédiaire possède les propriétés de codage caractéristiques du précurseur de IGF-I, n étant égal à 105.

4.  Procédé de production de plasmide codant pour un polypeptide mature possédant n amino-acides couplés à une séquence d'allongement d'amino-acides possédant m amino-acides, caractérisé en ce qu'un intermédiaire ayant la composition définie dans la revendication 1 est réuni par ligation au niveau du codon 1 à un segment synthétique de liaison possédant un codon d'initiation ATG conjointement avec un site de restriction Cla I à l'extrémité 5' du brin positif, ledit segment synthétique de liaison ayant la séquence

$$CGATG(XXX)_m$$
$$TAC(XXX)_m$$

dans laquelle X représente une base arbitraire, m est égal à 0 ou à un nombre entier, et les bases sont de préférence choisies de telle manière que la ou les premières bases dans le segment de liaison conjointement avec la ou les premières bases dans l'intermédiaire forment un site de restriction.

**5.** Procédé suivant la revendication 4, caractérisé en outre en ce qu'un promoteur et un site de liaison ribosomale convenables sont incorporés par ligation au plasmide.

**6.** Utilisation du plasmide produit suivant la revendication 4 ou 5 pour l'introduction dans un microorganisme, tel que E. coli, et la fermentation sur un substrat afin de produire le polypeptide désiré.

**Revendications pour l'Etat contractant suivant : AT**

**1.** Procédé de production d'un plasmide codant pour un polypeptide mature possédant n amino-acides couplés à une séquence d'allongement d'amino-acides possédant m amino-acides, dans lequel ledit plasmide contient un intermédiaire comprenant une séquence d'ADN qui consiste en deux fragments d'ADN couplés, dont le premier comprend la séquence codant pour les amino-acides 1 à n du polypeptide mature, suivie par un signal de terminaison de traduction, de préférence un site de restriction, et dont le second fragment d'ADN, qui est couplé au premier fragment d'ADN au niveau de la dernière base dans le premier fragment, contient un gène de résistance et une origine de réplication, caractérisé en ce que ledit intermédiaire est réuni par ligation à un segment synthétique de liaison au niveau du codon 1, ledit segment de liaison possédant un codon d'initiation ATG conjointement avec le site de restriction Cla I à l'extrémité 5' du brin positif, ledit segment de liaison ayant la séquence

$$\text{CGATG(XXX)}_m$$
$$\text{TAC(XXX)}_m$$

dans laquelle X représente une base arbitraire, m est égal à 0 ou à un nombre entier arbitraire, sous réserve que la ou les dernières bases dans le segment de liaison conjointement avec la ou les premières bases dans l'intermédiaire forment un site de restriction.

**2.** Procédé suivant la revendication 1, caractérisé en ce que le premier fragment d'ADN de l'intermédiaire possède les propriétés de codage caractéristiques de la hGH, n étant égal à 191.

**3.** Procédé suivant la revendication 1, caractérisé en ce que le premier fragment d'ADN de l'intermédiaire possède les propriétés de codage caractéristiques du précurseur de IGF-I, n étant égal à 105.

**4.** Procédé suivant l'une quelconque des revendications 1 à 3, caractérisé en outre en ce qu'un promoteur et un site de liaison ribosomale convenables sont incorporés par ligation au plasmide.

**5.** Utilisation du plasmide produit suivant l'une quelconque des revendications 1 à 4 pour l'introduction dans un micro-organisme, tel que E. coli, et la fermentation sur un substrat afin de produire le polypeptide désiré.

↓

-26
ATG GCT ACA GGC TCC CGG ACG TCC CTG CTC CTG GCT TTT GGC CTG CTC TGC CTG CCC TGG
MET ALA THR GLY SER ARG THR SER LEU LEU LEU ALA PGE GLY LEU LEU CYS LEU PRO TRP

START OF hGH

-6                                    ↓
CTT CAA GAG GGC AGT GCC TTC CCA ACC ATT CCC TTA TCC AGG CTT TTT GAC AAC GCT ATG
LEU GLN GLU LGY SER ALA PHE PRO THR ILE PRO LEU SER ARG LEU PHE ASP ASN ALA MET

15                                23  24
CTC CGC GCC CAT CGT CTG CAC CAG CTG GCC TTT GAC ACC TAC CAG GAG TTT GAA GAA GCC
LEU ARG ALA HIS ARG LEU HIS GLN LEU ALA PHE ASP THR TYR GLN GLU PHE GLU GLU ALA

35
TAT ATC CCA AAG GAA CAG AAG TAT TCA TTC CTG CAG AAC CCC CAG ACC TCC CTC TGT TTC
TYR ILE PRO LYS GLU GLN LYS TYR SER PHE LEU GLN ASN PRO GLN THR SER LEU CYS PHE

55
TCA GAG TCT ATT CCG ACA CCC TCC AAC AGG GAG GAA ACA CAA CAG AAA TCC AAC CTA GAG
SER GLU SER ILE PRO THR PRO SER ASN ARG GLU GLU THR GLN GLN LYS SER ASN LEU GLU

*Fig.1*

EP 0 218 651 B1

CTG CTC CGC ATC TCC CTG CTG CTC ATC CAG TCG TGG CTG GAG CCC GTG CAG TTC CTC AGG
LEU LEU ARG ILE SER LEU LEU LEU ILE GLN SER TRP LEU GLU PRO VAL GLN PHE LEU ARG

AGT GTC TTC GCC AAC AGC CTG GTG TAC GGC GCC TCT GAC AGC AAC GTC TAT GAC CTC CTA
SER VAL PHE ALA ASN SER LAU VAL TYR GLY ALA SER ASP SER ASN VAL TYR ASP LEU LEU

AAG GAC CTA GAG GAA GGC ATC CAA ACG CTG ATG GGG AGG CTG GAA GAT GGC AGC CCC CGG
LYS ASP LEU GLU GLU GLY ILE GLN THR LEU MET GLY ARG LEU GLU ASP GLY SER PRO ARG

ACT GGG CAG ATC TTC AAG CAG ACC TAC AGC AAG TTC GAC ACA AAC TCA CAC AAC GAT GAC
THR GLY GLN ILE PHE LYS GLN THR TYR SER LYS PHE ASP THR ASN SER HIS ASN ASP ASP

GCA CTA CTC AAG AAC TAC GGG CTG CTC TAC TGC TTC AGG AAG GAC ATG GAC AAG GTC GAG
ALA LEU LEU LYS ASN TYR GLY LEU LEU TYR CYS PHE ARG LYS ASP MET ASP LYS VAL GLU

ACA TTC CTG CGC ATC GTG CAG TEC CGC TCT GTG GAG GGC AGC TGT GGC TTC TAG CTG CCC
THR PHE LAU ARG ILE VAL GLN CYS ARG SER VAL GLU GLY SER CYS GLY PHE STOP

GGGTGGCATCCCTGTGACCCCTCCCCAGTGCCTCTCCTGG

*Fig.1*

EP 0 218 651 B1

*Fig.2*

*Fig.3*

Fig.4

*Fig. 5*

Fig.6

Fig. 7

DETAILED CUT OF HD-59-10

Fig. 8

Fig.9

Fig. 10

cDNA from GT 11

Fig. 11

GGCG CTG TGC CTG CTC ACC TTC ACC AGC TCT GOC ACG GCT GGA CCG
(I) (2) (3) (4)(5)

aal aa2
GGA CCG
Gly Pro

Fig. 12

CATCGATG GAA CCG
ClaI Met Gly Pro

Met-IGF I-precursor (106 aa)

pAT153 ClaI/BamHI

x ClaI linker

*Fig. 13*

ClaI    69  70  71                          HaeII

TCA GCT CGC TCT GTC CGT GCC CAG CGC CAC

ME AE

BamHI

+ synthetic linker

GCT TAG TA
CGA ATC ATT CGA

ClaI   aa70

Ala Stop Stop

GCT TAG TAA GCTT

ME AE

aa70

Hind III

*Fig. 14*